Europäisches Patentamt

(19) European Patent Office

Office européen des brevets

(11) Publication number: **0 070 634**
A2

(12) # EUROPEAN PATENT APPLICATION

(21) Application number: 82303371.7

(22) Date of filing: 28.06.82

(51) Int. Cl.³: **A 61 K 6/08,** C 08 F 2/50

(30) Priority: 15.07.81 US 283506

(43) Date of publication of application: 26.01.83
Bulletin 83/4

(84) Designated Contracting States: DE GB

(71) Applicant: DENTSPLY INTERNATIONAL INC., 570 W.
College Avenue P.O. Box 872, York
Pennsylvania 17405 (US)

(72) Inventor: Wang, Wu Lan, 45 South Horseshoe Drive,
Milford Delaware 19963 (US)

(74) Representative: Newens, Leonard Eric et al, F.J.
CLEVELAND CO. 40/43 Chancery Lane, London
WC2A 1JQ (GB)

(54) Visible light curable sealants.

(57) Visible light curable dental sealants are provided having improved color stability. According to a preferred embodiment, such materials are formulated from about 30% to about 60% by weight of particulate, inorganic fillers and a resin component comprising a binder resin, a diluent, and a two-component photosensitizing system comprising an alpha diketone and an amine reducing agent. Sealing processes employing such sealants are also disclosed.

EP 0 070 634 A2

## Background of the Invention

### Field of the Invention

This invention provides dental sealants and other coatings which are curable by the action of visible light. More particularly, formulations are disclosed which exhibit good cured strength, and which are easily used by the practitioner. Accordingly, such sealants are provided which may be cured in relatively thin films to yield hard, durable, cohesive, and well-adhering protective coatings, especially on teeth.

### Description of the Prior Art

Numerous dental sealants are known to those skilled in the art. Accordingly, it is known to apply to teeth liquid formulations which are capable of drying or setting to form a continuous coating on at least one surface of a tooth, which coating adheres tightly to that surface. Such coating is designed to be protective of the underlying tooth structure. Accordingly, it is necessary that this coating or sealant have high strength, be continuously cohesive, have limited water absorption, and be strongly bonded to the underlying surface.

In the past, most dental sealants have employed formulations which dry or cure by autocatalytic polymerization to form the sealant coating.  More recently, it has been known to provide polymerizable formulations which may be activated by ultraviolet light in such dental sealing systems.  Examples of such ultraviolet dental sealants are NUVA-SEAL® and NUVA-COTE® produced by the L.D. Caulk Division of Dentsply International Inc.  While these ultraviolet light curable dental sealants are preferred from the standpoint of ease of application and use, the employment of ultraviolet radiation in areas adjacent to the eyes and soft tissues is seen to be a significant detriment.  Accordingly, it is desired to provide dental sealant materials and other coatings which are curable entirely by exposure to light in the visible spectrum.  Attempts have been made to provide visible light curing dental sealant compositions.  Accordingly, the Kultzer & Co., GMBH has offered a material called DURAFIL BOND™ which reports to be curable by exposure to visible light.  It has been found, however, that exposure of DURAFIL BOND™ to light having no substantial component in wavelengths shorter than about 4,000 angstroms, such as with Dentsply International's PRISMA-LITE™ unit, does not result in acceptable polymerization.  Additionally, analysis of the light unit suggested for use in connection with the DURAFIL BOND™ product discloses that its output light comprises substantial portions in the ultraviolet region of the spectrum.  Accordingly, no sealant is known which is acceptably polymerizable employing solely visible light.  Certain visible light curable compositions useful as dental restoratives are known. See in this regard, U.S. Serial Nos. 182,626 and 182,624 assigned to the assignee of this invention. Use of such compositions as dental or other sealants is not suggested, however.

## Objects of the Invention

It is an object of this invention to provide dental sealant compositions which are polymerizable employing visible light. It is another object of this invention to provide dental sealant compositions which may be polymerized by exposure to light radiation having its wavelength components greater than about 4,000 angstroms. Yet another object is to provide such visible light curable dental sealants which, upon polymerization, are hard, durable, cohesive, and which adhere strongly to the surfaces of teeth. A still further object is to provide such dental sealants which remain in a fluid state until it is desired for polymerization to occur at which time the composition is hardened by exposure to visible light. Yet another object is to provide surface coatings which are curable by exposure to visible light, which coatings may be applied to a diverse range of objects to affect protection thereof from abrasion, impact, and other physical stresses. These and other objects are attained through employment of one or more embodiments of the present invention.

## Summary of the Invention

It has been found that visible light curable sealants may be formulated comprising from about 30% to about 60% of a particulate, inorganic filler, and from about 40% to about 70% of a polymerizable resin component comprising a binder resin, a diluent monomer, and a photosensitizing system comprising an alpha diketone, and an amine reducing agent. More particularly, it has been found that a critical range exists in the amount of particulate inorganic filler included in the visible light curable sealants according to this invention. It has been found that the average punch strength of cured specimens of the compositions of this invention varies with the amount of particulate, inorganic filler included therein. At the same time, the requirement of good manual workability on the part of dental practitioners places additional constraints upon the amount of filler to be used in conjunction with the visible light polymerizable materials of this invention. Accordingly, it has been found that amounts of filler of from about 30% to about 60% by weight are required to meet, at once, both of these requirements. It is even more preferred that amounts of filler of from about 40% to about 55% by weight be so included. It is also believed that sealants according to this invention having from about 30% to about 60% of filler exhibit superior surface curing characteristics.

## Detailed Description of the Invention

The visible light curable sealants of this invention comprise particulate, inorganic fillers, binder resins, diluent monomers, and a visible light photosensitizing system. Such blends may, optionally, include pigments, opacifiers, handling agents, and other modificants as will be appreciated by those skilled in the art.

Binder resins suitable for use in the practice of one or more of the embodiments of this invention include a wide variety of ethylenically unsaturated polymerizable compositions. Preferably, such resins are selected from the class of acrylated polyesters, polyethers and polyamides. Thus, the bis-glycidyl-methacrylate adduct of bisphenol A (bis-GMA) and its acrylic counterparts are preferred. Alternatively, the adducts of 2,2,-3-trimethylhexane diisocyanate with hydroxyethyl methacrylate, hydroxypropyl methacrylate, bis GMA, and other hydroxyalkyl acrylic species are also preferred. Those skilled in the art will appreciate that other acrylated polyesters, polyethers, etc. may also be suitable. Such acrylated materials may also be reacted with isocyanates to form urethanes useful as binder resins. Thus, bis-GMA may be reacted with a diisocyanate (or other isocyanate) such as hexamethylene diisocyanate, phenylene diisocyanate or a wide variety of other aliphatic and aromatic diisocyanates to provide useful binder resins. The adducts of bis-GMA with hexamethylene diisocyanate have been found to be the preferred binder resins presently known for use in this invention.

Diluent monomers may be any of a wide range of polymerizable monomers capable of sustaining a photochemically initiated polymerization. More preferably, such diluents may be the di-, tri- and higher acrylic esters of polyols such as ethylene glycol dimethacrylate, diethylene glycol dimethacrylate, triethylene glycol dimethacrylate, etc., trimethylolpropane trimethacrylate, analogous acrylates, and similar species.

The diluent monomers are added to the resin component of this invention, which comprises the binder resin and photosensitizing system, in amounts sufficient to attain the rheological properties desired in the final dental sealant composition in conjunction with the beneficial physical characteristics of the light cured sealant imparted by the particulate, inorganic filler component. Accordingly, diluent monomer should be included in the resin component of the compositions of this invention in an amount such that the resulting visible light curable dental sealant has a viscosity of from about 35,000 centipoises to about 70,000 centipoises. It is more preferred that the sealant have a viscosity of from about 45,000 centipoises to about 60,-000 centipoises and even more preferred that the viscosity be from about 50,000 to about 56,000 centipoises. Those skilled in the art will recognize that the foregoing viscosity ranges are representative of those dental sealant viscosities which are preferred by practitioners for convenient use. More particularly, diluent monomer is employed in the resin component in amounts of from about 5% to about 45% by weight of that component. It is preferred that from about 15% to about 40% and even more preferred that from about 25% to about 35% be included. Those skilled in the art will appreciate, however, that the best measure of the relative proportions of binder resin and diluent monomer is related to the apparent viscosity of the dental sealants to be thus formulated.

For the formulation of the visible light polymerizable sealant compositions of this invention, the binder resin, diluent monomer and visible light photosensitizing system comprising the resin component are blended together with from about 40% to about 60% of particulate, inorganic pigments and other modificants. Those skilled in the art will appreciate that the amount of filler loading which may be accomplished with a given resinous system will depend upon several variables including the identity of the resins and fillers and the particle sizes of the fillers. Among those fillers which are especially suited for use in the practice of this invention are inorganic glasses. Preferred among these are barium aluminum silicate, lithium aluminum silicate, strontium, lanthanum, tantalum, etc., glasses, and related materials. Silica, especially in submicron sizes, quartz, and other fillers may also be employed in some formulations. Mixtures of fillers is often preferred. Such fillers are preferably silanated prior to use in the restoratives of this invention. Silanation is well known to those skilled in the art and any effective silanating compound known to them may be used for this purpose.

Fillers are selected having particle sizes which are, in general, less than about 50 microns. It is known that smaller sized filler particles result in highly polishable dental materials, but that the concomitant increase in surface area diminishes the overall filler loading possible with a given resin. Such lower loadings may be manifested by lesser degrees of strength, hardness, and durability in the resulting polymerized structures. It is possible to employ submicron sized fillers in some cases, however. The ratio of resin to filler employed for the practice of this invention must take account of the filler size as suggested above.

Amounts of particulate, inorganic filler are added to the compositions of this invention in amounts of from about 30% to about 60% by weight. It is preferred that amounts of from about 45% to about 55% by weight be added. It has been found that for a given resin-filler system, strength of polymerized layers of sealant increases with increased loading of filler to an optimum at which point the strength begins to decrease. It is not possible to employ optimum amounts of filler from the point of view of strength in these systems, however, in all instances. Thus, systems employing filler in amounts such that the strength of the resulting, polymerized materials is optimized do not have the necessary viscosity and other workability parameters which are necessary for good workability by the practitioner. Accordingly, it has been determined that the dental sealants of the present invention must have, at the same time, amounts of particulate, inorganic filler ranging from about 30% to about 60% while the overall compositions maintain viscosities of from about 35,000 centipoises to about 70,000 centipoises. It is preferred that the compositions employ amounts of filler of from about 45% to about 55% by weight and that they have viscosities of from about 45,000 to about 60,000 centipoises. It is even more preferred that they have viscosities in the range of from about 50,000 to about 56,000 centipoises for optimum suitability and use by the dental practitioner.

The photosensitizing system employed in the formulation of dental materials according to the practice of this invention comprises two components, an alpha diketone photosensitive species (also known as an alpha, beta diketone) and an amine reducing agent. While any alpha diketone which is capable of initiating polymerization in the polymerizable systems of this invention may be employed, camphoroquinone, benzil, biacetyl, 9,10-phenanthrenequinone, and naphthoquinone have been found to be preferred. Most preferred is camphoroquinone.

The alpha diketone is combined with an amine reducing agent; the two taken together form the visible light sensitizing system useful for the practice of this invention. Numerous amines have been found to be useful as reducing agents for the alpha diketones used herein. Thus, amines such as tributylamine, tripropylamine are useful. Still more useful are substituted amines such as N-alkyl dialkanolamines and trialkanolamines. N-methyldiethanolamine is most preferred. Those skilled in the art will appreciate that numerous other alpha diketones and amine reducing agents may be employed without deviating from the spirit of this invention.

The amounts of the components of the visible light sensitizing system of this invention are selected to be such that substantial polymerization of the dental sealants of this invention occurs upon exposure to visible light. The amount of alpha diketone photosensitizer comprises from about 0.05% to about 0.50% by weight based upon the total weight of the binder resin, diluent monomer and photosensitizing system. It is even more preferred to employ from about 0.10% to about 0.30% of an alpha diketone. The amount of amine reducing agent is less critical. It is useful to employ from about 0.2% to about 1.0% of an amine with from about 0.3% to about 0.6% being preferred, based on the total weight of binder, diluent and photosensitizing system.

While the components of the polymerized blend may be added in any order, it has been found useful and convenient to mix the binder resin and diluent together, to add the photosensitizing system components, and then to blend in the filler together with pigments and other modifying agents. In practice, the binder resin and diluent are mixed together in a proportion such that the final, filled, polymerizable composition will have the desired viscosity.

The methods of use of the visible light curable dental sealants according to this invention follow, to an extent, those currently practiced by those skilled in the art in employing conventional sealants. Thus, the surface to which sealant is to be applied is cleansed of decayed material and, preferably, etched with acid to promote bonding. The visible light curable sealants according to this invention, are then applied to the prepared surface and brushed or otherwise smoothed into a continuous, homogenous, uniform coating. The need for proper viscosity of the sealant compositions is most apparent from consideration of this step. By virtue of the fact that the sealants according to this invention remain in a liquid state until they are caused to polymerize through exposure to a visible light source, they may be applied at leisure by the practitioner who need not worry that premature setting of the composition will occur. When a satisfactory coverage has been attained, the visible light curable materials of this invention are then irradiated with visible light for a period of time sufficient to cause substantial polymerization thereof. It is preferred to use a visible light source which excludes all or substantially all ultraviolet components. In this regard, wavelengths shorter than about 4,000 angstroms are considered to be ultraviolet. A preferred means for securing visible light irradiation is through the employment of a PRISMA-LITE™ polymerization unit available from the L.D. Caulk Division of Dentsply International Inc. With certain formulations, it is possible to obtain a thin, sticky film on an exterior surface of the polymerized sealant. If this occurs, it is a simple matter to remove it with, for example, a damp cotton roll to present a clean, hard, durable surface.

## Example I

A resin component was formulated from 66.0 grams of the hexamethylene diisocyanate adduct of bis-GMA, 18 grams of bis-GMA, 15.0 grams of triethyleneglycol dimethacrylate, 0.035 grams of butylated hydroxy toluene, 0.153 grams of camphoroquinone, and 0.494 grams of methyl diethanolamine. To this resin component was added, in separate batches, varying amounts of a 50:50 blend by weight of ground and silanated barium glass (RAYSORB™ T-3000) and lithium glass (Cer-Vit™ T1000, Owens-Corning Fiberglas Corp.) in amounts such that the inorganic, particulated glass comprised 0%, 20%, 40%, 50%, and 60% by weight of the total, filled, polymerizable material. Disc-shaped specimens approximately 1 millimeter thick by 10 millimeters in diameter were prepared from the above compositions by curing them in a brass ring for approximately 10 seconds exposure to visible light (400 to 500 NM, 0.4 millowatts/cm$^2$/sec. at contact) from a PRISMA-LITE™ unit from the L.D. Caulk Division of Dentsply International Inc. The polymerized specimens were tested for strength employing an Instron testing unit. Each disc was supported by a metal plate having a hole therein. A 1 millimeter spindle designed so as to cooperate with the hole, was caused to exert force upon the specimen until breakage. The force to break the specimen was recorded and interpreted in terms of load per unit area expressed in meganewtons per square meter (Mnwt/m$^2$). Averages over at least three separate samples are recorded in the following Table I.

TABLE I

| % FILLER | AVERAGE PRESS STRENGTH (Mnwt/m$^2$) | STANDARD DEVIATION | NO. SAMPLES |
|---|---|---|---|
| 0 | 17.8 | 3.0 | 5 |
| 20 | 43.9 | 4.6 | 3 |
| 40 | 45.9 | 8.2 | 9 |
| 50 | 36.5 | 3.2 | 5 |
| 60 | 23.4 | 7.6 | 3 |

# C L A I M S

1. A dental sealant curable by irradiation with visible light comprising:

from about 30% to about 60% by weight of a particulate, inorganic filler, and

from about 40% to about 70% of a polymerizable resin component comprising:

a binder resin,

a diluent monomer, and

a photosensitizing system comprising:

an alpha diketone, and

an amine reducing agent,

said photosensitizing system being present in an amount sufficient to cause substantial polymerization of said sealant upon irradiation with visible light.

2. The sealant of claim 1 wherein said binder resin is selected from the group consisting of acrylated polyesters, acrylated polyesters reacted with isocyanates, and hydroxyalkyl acrylic species reacted with isocyanates, said alpha diketone is selected from the group consisting of camphoroquinone, benzil, biacetyl, 9,10-phenanthrenequinone, and naphthoquinone, said amine is either N-alkyl dialkanolamine or trialkanolamine, and said fillers are selected from the group consisting of inorganic glasses and silica.


3. The sealant of claim 1 or 2 wherein said alpha diketone is present in an amount of from about 0.05% to about 0.30% based upon the total weight of said binder resin, diluent and photosensitizing system.


4. The sealant of claim 1 wherein said filler is present in an amount of from about 45% to about 55% by weight.


5. The sealant of claim 1 having a viscosity between about 35,000 and about 70,000 centipoises.


6. A process of sealing teeth comprising:

applying to a prepared tooth a dental sealant curable by irradiation with visible light comprising:

from about 30% to about 60% of a particulate, inorganic filler, and

a polymerizable resin component comprising:

a binder resin,

a diluent monomer, and

a photosensitizing system comprising:

an alpha diketone, and

an amine reducing agent,

said photosensitizing system being present in an amount sufficient to cause substantial polymerization of said composition upon irradiation with visible light, and .

exposing said composition to visible light to effect substantial polymerization thereof.

7. The process of claim 6 wherein said binder resin is selected from the group consisting of acrylated polyesters, acrylated polyesters reacted with isocyanates, and
hydroxyalkyl acrylic species reacted with isocyanates, said
alpha diketone is selected from the group consisting of camphoroquinone, benzil, biacetyl, 9,10-phenanthrenequinone, and naphthoquinone, said amine is either N-alkyl dialkanolamine or
trialkanolamine, and said fillers are selected from the group
consisting of inorganic glasses and silica.

8. The process of claim 6 or 7 wherein said alpha
diketone is present in an amount of from about 0.05% to
about 0.30% based upon the total weight of said binder resin, diluent, and photosensitizing system.

9. The process of claim 6 or 7 wherein said light
is composed predominantly of wavelengths longer than about
4,000 angstroms.

0070634

10. The process of claim 6 wherein said filler is present in amount of from about 45% to about 55% by weight.

11. The process of claim 6 wherein said sealant has a viscosity of from about 35,000 to about 70,000 centi-poises.